# EUROPEAN PATENT SPECIFICATION

(11) **EP 2 997 961 B1**
(45) Date of publication and mention of the grant of the patent: **01.03.2017**
(21) Application number: 14798170.8
(22) Date of filing: 01.05.2014
(51) Int. Cl.: A61K 8/73, A61K 8/34, A61K 8/36, A61K 8/81, A61Q 19/10, C11D 3/12, C11D 3/20, C11D 3/37, A61K 8/02, C11D 3/22

(54) **SKIN CLEANSING AGENT**
HAUTREINIGUNGSMITTEL
PRODUIT NETTOYANT POUR LA PEAU

(30) Priority: 14.05.2013 JP 2013101911
(43) Date of publication of application: 23.03.2016
(73) Proprietor: Fujifilm Corporation, Minato-ku Tokyo 106-8620 (JP)
(72) Inventor: AIMI, Makiko, Ashigarakami-gun Kanagawa 258-8577 (JP)
(74) Representative: Klunker . Schmitt-Nilson . Hirsch
(86) International application number: PCT/JP2014/062089
(87) International publication number: WO 2014/185284

(56) References cited:
- EP-A2- 0 047 033
- WO-A1-2012/123157
- JP-A- S5 462 328
- JP-A- H03 227 914
- JP-A- S59 210 009
- JP-A- 2010 270 085
- JP-A- 2010 270 085
- JP-A- 2012 126 699
- JP-A- 2012 126 699
- ASAHI CHEMICAL INDUSTRY CO., LTD.: 'Genryo Shokai Kessho Cellulase (Avicel, Ceolus) no Keshohin eno Oyo' FRAGRANCE JOURNAL vol. 28, no. 7, 15 July 2000, pages 93 - 96, XP008178554

## Description

### BACKGROUND OF THE INVENTION

### 1. Field of the Invention

The present invention relates to a skin cleansing agent.

### 2. Description of the Related Art

In a skin cleansing agent, a higher fatty acid salt is frequently used because it results in excellent detergency and makes the skin cleansing agent feel fresh to the touch at the time of use.

However, the higher fatty acid salt results in insufficient foamability at the time of cleansing and leaves a feeling of tightness on the skin after cleansing. Furthermore, if the skin cleansing agent contains the higher fatty acid salt, the preservation stability of the preparation has problems such as the precipitation of the higher fatty acid salt during the preservation at a low temperature, the hardening of the skin cleansing agent, and the phase separation that occurs during the preservation at a high temperature.

Various studies on how to solve these problems have been performed.

For example, JP1991-227914A (JP-H03-227914A) describes a liquid detergent composition in which a higher fatty acid salt is mixed with a cationic polymer and a glucose derivative so as to improve the foamability.

JP2010-180181 A describes a detergent composition in which a higher fatty acid salt is mixed with two kinds of polyol so as to improve a feeling of tightness after cleansing.

JP2012-167035A describes a cream-type skin cleansing agent in which a higher fatty acid salt is mixed with a nonionic surfactant and oil-absorbing powder so as to improve the foamability and a feeling of tightness after cleansing.

JP2008-37841A describes a soap composition in which a higher fatty acid salt is mixed with crystalline cellulose having different average particle sizes such that the soap composition exhibits excellent foamability at the time of use and brings about an appropriate scrubbing effect.

JP2010-270085A describes a skin cleansing agent in which a higher fatty acid salt is mixed with crystalline cellulose and a cellulose-based thickener so as to obtain a scrubbing effect and to improve the preservation stability of the preparation.

### SUMMARY OF THE INVENTION

Meanwhile, it has become clear that the cationic polymer mixed for improving the foamability contaminates a container because the cationic polymer exhibits spinnability in which it is drawn out into threads at the time of use. Furthermore, the crystalline cellulose, which brings about a scrubbing effect and has a large average particle size, may cause skin irritation or a foreign body sensation. In addition, it has become clear that, in any of the skin cleansing agents described in the published gazettes described above, the improvement of a feeling of tightness of the skin after cleansing or the improvement of the temporal stability is insufficient.

Under the current circumstances described above, the development of a skin cleansing agent, which is mild on the skin, exhibits excellent foamability, does not leave a feeling of tightness on the skin after cleansing, and exhibits excellent temporal stability, is awaited.

An object of the present invention is to provide a skin cleansing agent which is mild on the skin, exhibits excellent foamability, is inhibited from leaving a feeling of tightness on the skin after cleansing and inhibited from contaminating a container by being drawn out into threads at the time of use, and exhibits excellent temporal stability.

The means for achieving the aforementioned object is disclosed in the claims.

According to the present invention, it is possible to provide a skin cleansing agent which is mild on the skin, exhibits excellent foamability, is inhibited from leaving a feeling of tightness on the skin after cleansing and inhibited from contaminating a container by being drawn out into threads at the time of use, and exhibits excellent temporal stability.

### DESCRIPTION OF THE PREFERRED EMBODIMENTS

Hereinafter, the skin cleansing agent of the present invention will be specifically described.

In the present specification, a range of numerical values described by using "to" is a range that includes numerical values listed before and after "to" as a minimum value and a maximum value respectively.

In the present specification, when there is plurality of substances corresponding to each of the components in a composition, unless otherwise specified, the amount of each of the components in the composition means the total amount of the plurality of substances present in the composition.

In the present specification, the "temporal stability" means a property in which the change in the hardness of the skin cleansing agent is inhibited even over a passage of time.

### <Skin cleansing agent>

The skin cleansing agent of the present invention contains crystalline cellulose having an average particle size of equal to or less than 100 µm, polyquaternium-7, a higher fatty acid salt having 8 to 22 carbon atoms, and a polyol. If necessary, the skin cleansing agent of the present invention may further contain other components.

In the skin cleansing agent of the present invention, a higher fatty acid salt having 8 to 22 carbon atoms, a polyol, crystalline cellulose having an average particle size of equal to or less than 100 µm, and polyquaternium-7 are concurrently used. Accordingly, the skin cleansing agent is mild on the skin, exhibits excellent foamability, does not leave a feeling of tightness on the skin after cleansing, and exhibits excellent temporal stability.

The crystalline cellulose having an average particle size of equal to or less than 100 µm is considered to inhibit the spinnability resulting from a cationic polymer by being incorporated into diallyldimethylammonium chloride (DADMAC) structures, which are partial structures of the polyquaternium-7 as the cationic polymer.

### (Crystalline cellulose)

The skin cleansing agent of the present invention contains crystalline cellulose having an average particle size of equal to or less than 100 µm.

The crystalline cellulose is designated by CAS No 9004-34-6. It is a component obtained by partially depolymerizing α-cellulose, which is obtained in the form of pulp from fibrous plants, by using a mineral acid and purifying the resultant.

The size and shape of the crystalline cellulose vary with the processing method. In the skin cleansing agent of the present invention, any crystalline cellulose can be used without particular limitation as long as it has an average particle size of equal to or less than 100 µm.

The average particle size of the crystalline cellulose used in the skin cleansing agent of the present invention is equal to or less than 100 µm.

It is not preferable for the average particle size of the crystalline cellulose to be greater than 100 µm, because a user experiences skin irritation or has a foreign body sensation, or the temporal stability of the skin cleansing agent deteriorates.

From the viewpoint of inhibiting the spinnability of the skin cleansing agent and improving the temporal stability of the skin cleansing agent, the average particle size of the crystalline cellulose is preferably 20 µm to 100 µm, more preferably 50 µm to 90 µm, and even more preferably 50 µm.

In the present specification, for the average particle size of the crystalline cellulose, the catalogue values of raw materials are referred to.

The shape of the crystalline cellulose may be any of a fibrous shape, a spherical shape, a plate shape, and an amorphous shape. Among these, from the viewpoint of improving the foamability, the foam retainability, and the foam quality of the skin cleansing agent, the crystalline cellulose preferably has a fibrous shape.

More specifically, regarding the fibrous shape which is a preferred embodiment of the shape of the crystalline cellulose, when a major axis and a minor axis of the fibrous crystalline cellulose are measured, and a ratio of the major axis to the minor axis (ratio L/D), which is a ratio between the major axis and the minor axis, is calculated, the ratio L/D is preferably equal to or greater than 1.5, more preferably equal to or greater than 2, and even more preferably equal to or greater than 2.8.

Provided that the length of the crystalline cellulose in a long-axis direction is L, and the length of the crystalline cellulose in a short-axis direction is D, the ratio of the major axis to the minor axis of the crystalline cellulose is represented by L/D. The ratio of the major axis to the minor axis of the crystalline cellulose is a value of the arithmetic mean of a ratio L/D that is determined by magnifying the crystalline cellulose molecules with a microscope and measuring the length L in a long-axis direction and the length D in a short-axis direction of each of 10 randomly selected crystalline cellulose molecules.

Each of the crystalline cellulose molecules observed is interposed between two parallel lines A and A' such that the crystalline cellulose molecule comes into contact with the lines A and A'. At this time, when the interval between A and A' becomes maximum, the distance between A and A' is taken as the length of the crystalline cellulose in the long-axis direction. Furthermore, each of the crystalline cellulose molecules is interposed between two parallel lines B and B', which are perpendicular to the two parallel lines A and A' for determining the length of the crystalline cellulose in the long-axis direction, such that the crystalline cellulose molecule comes into contact with the two parallel lines B and B'. At this time, the distance between B and B' is taken as the length of the crystalline cellulose in the short-axis direction.

The bulk density of the crystalline cellulose is preferably 0.1 to 0.5, more preferably 0.1 to 0.43, and even more preferably 0.12 to 0.21.

The bulk density of the crystalline cellulose can be measured according to the method described in the 16^{th} revised edition of The Japanese Pharmacopoeia.

As the crystalline cellulose, commercially available products can also be used. Examples of the commercially available products include Ceolus (registered trademark) KG-802 and 1000; Ceolus pH-101, 101D, 102, 200, 301, 301D, 302, and F20JP; Ceolus UF-702 and 711 (trade name, all manufactured by Asahi Kasei Chemicals Corporation); and the like.

One kind of any of these crystalline celluloses may be used singly, or two or more kinds thereof may be used concurrently.

From the viewpoint of inhibiting the spinnability and improving the temporal stability, the content of the crystalline cellulose is preferably 0.01% by mass to 10% by mass, more preferably 0.01% by mass to 5% by mass, and even more preferably 0.05% by mass to 1% by mass, with respect to the total mass of the skin cleansing agent.

### (Polyquaternium-7)

The skin cleansing agent of the present invention contains polyquaternium-7.

Polyquaternium-7 is designated by CAS No. 26590-05-6. It is a polymer of acrylic acid amide and a quaternary ammonium salt obtained from dimethyldiallylammonium chloride. Polyquaternium-7 is a compound included in cationic polymers.

As the polyquaternium-7, commercially available products can also be used. Examples of the commercially available products include MERQUAT 550 (trade name, The Lubrizol Corporation), Lipoflow MN (trade name, Lion Corporation), and the like.

From the viewpoint of improving the foam retainability, the foam quality, and the feeling of moistness of the skin after cleansing, the content of the polyquaternium-7 is preferably 0.01% by mass to 5% by mass, more preferably 0.05% by mass to 3% by mass, and even more preferably 0.1% by mass to 1% by mass, with respect to the total mass of the skin cleansing agent.

The content ratio of the crystalline cellulose to the polyquaternium-7 is preferably 1:5 to 20:1 (crystalline cellulose:polyquaternium-7) based on mass. If the content ratio of the crystalline cellulose to the polyquaternium-7 is within the aforementioned range, it is possible to effectively inhibit the spinnability of the skin cleansing agent that results from the polyquaternium-7.

The content ratio of the crystalline cellulose to the polyquaternium-7 is more preferably 1:2 to 10:1 (crystalline cellulose:polyquaternium-7) based on mass.

### (Higher fatty acid salt)

The skin cleansing agent of the present invention contains a higher fatty acid salt having 8 to 22 carbon atoms (hereinafter, referred to as a "higher fatty acid salt").

The higher fatty acid salt is constituted with an aliphatic hydrocarbon group, a carboxyl group, and a base.

The aliphatic hydrocarbon group just needs to be an aliphatic hydrocarbon group having 7 to 21 carbon atoms, and may be any of a saturated or unsaturated hydrocarbon group and a linear or branched hydrocarbon group.

Specifically, examples of the higher fatty acid include lauric acid, myristic acid, palmitic acid, stearic acid, arachidonic acid, behenic acid, isostearic acid, oleic acid, 2-palmitoleic acid, petroselinic acid, elaidic acid, ricinoleic acid, linoleic acid, linoelaidic acid, linolenic acid, coconut oil fatty acid, palm kernel oil fatty acid, beef tallow fatty acid, and the like.

Examples of the base, which is a counterion of the higher fatty acid in the higher fatty acid salt, include bases like an inorganic base such as sodium, potassium, or magnesium; ammonium; alkanolamines such as monoethanolamine, diethanolamine, triethanolamine, 2-amino-2-methylpropanol, or 2-amino-2-methylpropanediol; and basic amino acids such as lysine or arginine. Among these, the inorganic base of an alkali metal such as sodium or potassium is preferable.

The higher fatty acid salt may be contained as a fatty acid salt in the skin cleansing agent. Alternatively, a fatty acid and a basic component may be separately contained in the skin cleansing agent and form a fatty acid salt in the composition.

As the combination of the higher fatty acid salt and the base, a combination of myristic acid and potassium, a combination of palmitic acid and potassium, a combination of stearic acid and potassium, and a combination of lauric acid and potassium are preferable.

One kind of any of the higher fatty acid salts may be used singly, or two or more kinds thereof may be used concurrently.

From the viewpoint of improving the foamability and the temporal stability, the content of the higher fatty acid salt is preferably 15% by mass to 60% by mass, more preferably 20% by mass to 50% by mass, and even more preferably 25% by mass to 35% by mass, with respect to the total mass of the skin cleansing agent.

### (Polyol)

The skin cleansing agent of the present invention contains a polyol.

As the polyol, any polyol can be used without particular limitation as long as it has two or more hydroxyl groups.

Examples of the polyol include glycerin, diglycerin, triglycerin, polyglycerin, 3-methyl-1,3-butanediol, 1,3-butylene glycol, isoprene glycol, polyethylene glycol, 1,2-pentanediol, 1,2-hexanediol, propylene glycol, dipropylene glycol, ethylene glycol, diethylene glycol, neopentyl glycol, propanediol, glucoside, glyceryl glucoside, trehalose, glycosyl trehalose, xylitol, fructose, lactose, trimethylglycine, sorbitol, maltitol, and the like.

Among these polyols, from the viewpoint of improving the feeling of moistness after cleansing and improving the temporal stability, glycerin, 1,3-butylene glycol, polyethylene glycol having an average molecular weight of equal to or less than 10,000, glycosyl trehalose, sorbitol, and glyceryl glucoside are preferable.

As the polyol, commercially available products can also be used. Examples of the commercially available products include PEG-6, 20, and 32 from NOF CORPORATION, glycerin from New Japan Chemical Co., Ltd., and the like.

One kind of any of the polyols may be used singly, or two or more kinds thereof may be used concurrently.

From the viewpoint of improving the feeling at the time of use and improving the temporal stability, the content of the polyol is preferably 5% by mass to 60% by mass, more preferably 10% by mass to 50% by mass, and even more preferably 15% by mass to 50% by mass, with respect to the total mass of the skin cleansing agent.

### (Other components)

If necessary, the skin cleansing agent of the present invention may contain components that are known as additives for a skin cleansing agent, as long as the effects of the present invention are not impaired. Examples of components that are preferable as additives for a skin cleansing agent include clay, a nonionic surfactant, a conditioning agent, and the like.

### (Clay)

The skin cleansing agent of the present invention preferably further contains clay. If the skin cleansing agent contains clay, the detergency thereof is further improved.

The clay means a component industrially purified from natural minerals which contain hydrated aluminum silicate as a main component. The clay is preferably a component which exhibits at least either a physical property of oil absorbency or a physical property of water-swellability.

Specifically, examples of the clay include kaolin, talc, mica, bentonite, montmorillonite, saponite, hectorite, and the like.

Among these, from the viewpoint of improving the detergency and from the viewpoint of the versatility of clay as a raw material, kaolin or talc is preferable as the clay.

As the clay, commercially available products can also be used. Examples of the commercially available products include kaolin (trade name: Color Clay series, MATSUMOTO TRADING Co., Ltd.), kaolin (trade name: 2747 Kaolin USP BC, INA TRADING CO., LTD.), talc (trade name: Talc JA series, ASADA MILLING CO., LTD.), and the like.

One kind of any of these clays may be used singly, or two or more kinds thereof may be used concurrently.

When the skin cleansing agent of the present invention contains clay, from the viewpoint of further improving the detergency and the temporal stability, the content of the clay is preferably 0.01% by mass to 10% by mass, more preferably 0.05% by mass to 5% by mass, and even more preferably 0.1% by mass to 2% by mass, with respect to the total mass of the skin cleansing agent.

### (Nonionic surfactant)

The skin cleansing agent of the present invention preferably further contains a nonionic surfactant having a linear fatty acid ester structure having 8 to 22 carbon atoms (hereinafter, referred to as a "nonionic surfactant"). If the skin cleansing agent contains the nonionic surfactant, the foamability and the temporal stability thereof are further improved.

The nonionic surfactant preferably used in the skin cleansing agent of the present invention has a linear fatty acid ester structure having 8 to 22 carbon atoms.

Specifically, examples of the linear fatty acid having 8 to 22 carbon atoms include lauric acid, myristic acid, palmitic acid, stearic acid, behenic acid, oleic acid, linoleic acid, and the like.

Among these, from the viewpoint of improving the temporal stability, a nonionic surfactant having an ester structure of a linear fatty acid selected from stearic acid, palmitic acid, myristic acid, and lauric acid is preferable.

As the nonionic surfactant, commercially available products can also be used. Examples of the commercially available products include glyceryl stearate (trade name: NIKKOL MGS series, Nikko Chemicals Co., Ltd.), glyceryl myristate, (trade name: NIKKOL MGM, Nikko Chemicals Co., Ltd.), glyceryl palmitate (trade name: EMALEX GMS-P, Nihon Emulsion Co., Ltd.), and the like.

One kind of any of these nonionic surfactants may be used singly, or two or more kinds thereof may be used concurrently.

When the skin cleansing agent of the present invention contains a nonionic surfactant, from the viewpoint of improving the foamability and the temporal stability, the content of the nonionic surfactant is preferably 0.01% by mass to 10% by mass, more preferably 0.1% by mass to 5% by mass, and even more preferably 0.5% by mass to 2% by mass, with respect to the total mass of the skin cleansing agent.

### (Conditioning agent)

The skin cleansing agent of the present invention preferably further contains a conditioning agent. If the skin cleansing agent contains the conditioning agent, the feeling of moistness after cleansing is further improved.

The conditioning agent means a component useful for bringing about a conditioning effect (emollient effect) which keeps the skin moist and softens the skin.

The conditioning agent is not particularly limited as long as it can bring about a conditioning effect, and any of a water-soluble conditioning agent and an oil-soluble conditioning agent can be used.

Examples of the water-soluble conditioning agent include urea, guanidine, hyaluronic acid, an aloe vera component, chitin, and the like.

Examples of the oil-soluble conditioning agent include mineral oil, petrolatum, branched hydrocarbons having 7 to 40 carbon atoms, an ester of a carboxylic acid having 1 to 30 carbon atoms and an alcohol having 1 to 30 carbon atoms, an ester of a dicarboxylic acid having 2 to 30 carbon atoms and an alcohol having 1 to 30 carbon atoms, a monoglyceride of a carboxylic acid having 1 to 30 carbon atoms, a diglyceride of a carboxylic acid having 1 to 30 carbon atoms, a triglyceride of a carboxylic acid having 1 to 30 carbon atoms, an ethylene glycol monoester of a carboxylic acid having 1 to 30 carbon atoms, an ethylene glycol diester of a carboxylic acid having 1 to 30 carbon atoms, a propylene glycol monoester of a carboxylic acid having 1 to 30 carbon atoms, a propylene glycol diester of a carboxylic acid having 1 to 30 carbon atoms, monoesters and polyesters of sugars and carboxylic acids having 1 to 30 carbon atoms, a polydialkyl siloxane, a polydiaryl siloxane, an alkaline polysiloxane, a cyclomethicone having 3 to 9 silicon atoms, an alkyl ether of polypropylene glycol that has 4 to 20 carbon atoms, a dialkyl ether having 8 to 30 carbon atoms, a phytosteryl ester, cholesteryl ester, jojoba seed oil, shea oil and fat, macadamia nut oil, Vaseline, and the like.

As the phytosteryl ester, phytosteryl isostearate, di(phytosteryl/octyldodecyl) N-lauroyl glutamate, phytosteryl hydroxystearate, and the like are preferable.

Among the aforementioned conditioning agents, from the viewpoint of improving the foam retainability, the feeling of moistness after cleansing, and the temporal stability, phytosteryl isostearate, di(phytosteryl/octyldodecyl) lauroyl glutamate, jojoba seed oil, shea oil and fat, macadamia nut oil, and Vaseline are more preferable. Furthermore, from the viewpoint of improving the temporal stability, phytosteryl isostearate and shea oil and fat are even more preferable.

As the conditioning agent, commercially available products can also be used. Examples of the commercially available products include phytosteryl isostearate (trade name: Phytosteryl isostearate, Tama Kagaku Kogyo Co., Ltd.), shea oil and fat (trade name: Shea Oil, INA TRADING CO., LTD.), and the like.

One kind of any of these conditioning agents may be used singly, or two or more kinds thereof may be used concurrently.

When the skin cleansing agent of the present invention contains a conditioning agent, from the viewpoint of improving the feeling of moistness after cleansing and improving the temporal stability, the content of the conditioning agent is preferably 0.01% by mass to 10% by mass, more preferably 0.1% by mass to 5% by mass, and even more preferably 0.1% by mass to 2% by mass, with respect to the total mass of the skin cleansing agent.

If necessary, the skin cleansing agent of the present invention may contain other components which are known as additives for a skin cleansing agent, in addition to the clay, the nonionic surfactant, and the conditioning agent which are preferable as additives for a skin cleansing agent. Examples of other components which are known as additives for a skin cleansing agent include components which can function as a moisturizing component, a cleansing component other than the higher fatty acid salt, an anti-inflammatory component, a bactericidal component, a colorant, an antioxidant, a preservative, and the like. A single component may perform two or more kinds of function.

The moisturizing component just needs to be a component which can function as a moisturizing component. Preferred examples thereof include hydrolyzed and hydrogenated starch, a fermented broth obtained by lactobacillus, a fermented broth of European pear juice, a blackberry lily extract, a bilberry leaf extract, an artichoke leaf extract, and the like.

The cleansing component other than the higher fatty acid salt just needs to be a component which can function as a cleansing component. Examples thereof include sodium methyl cocoyl taurate, sodium lauroyl glutamate, and the like.

The anti-inflammatory component just needs to be a component which can function as an anti-inflammatory component. Examples thereof include stearyl glycyrrhetinate, β-glycyrrhizic acid, glycyrrhetinic acid, allantoin, tranexamic acid, and the like.

The bactericidal component just needs to be a component which can function as a bactericidal component. Examples thereof include salicylic acid, sulfur, isopropyl methylphenol, and the like.

Examples of the colorant include titanium oxide, caramel, iron oxide, sodium copper chlorophyllin, and the like.

Examples of the antioxidant include tocopherol and derivatives thereof, ascorbic acid and derivatives thereof, astaxanthin, and the like.

Examples of the preservative include paraben, propylparaben, phenoxyethanol, ethylenediaminetetraacetic acid (EDTA), and the like.

If necessary, the skin cleansing agent of the present invention may contain, as other components, tocopherol acetate, pantothenyl alcohol, pyridoxine hydrochloride, nicotinic acid amide, urea, arbutin, ascorbyl glucoside, magnesium ascorbyl phosphate, and the like.

For example, the content of each of the aforementioned other components is preferably 0.001% by mass to 10% by mass, more preferably 0.01% by mass to 5% by mass, and even more preferably 0.1 % by mass to 3% by mass, with respect to the total mass of the skin cleansing agent.

The skin cleansing agent of the present invention is preferably used as a facial cleansing agent or body cleansing agent.

The manufacturing method of the skin cleansing agent of the present invention is not particularly limited, and the skin cleansing agent can be manufactured according to known methods. For example, the skin cleansing agent of the present invention can be obtained by mixing essential components with optional components, which can be concurrently used if necessary, by using a stirrer, an impeller stirrer, a homomixer, or the like.

### Examples

Hereinafter, the present invention will be more specifically described based on examples. The embodiments described below are examples of the constitution of the present invention, and the present invention is not limited to the following embodiments. In the following description, unless otherwise specified, the numerical values represented by using "%" are based on mass.

### [Examples 1 to 21 and Comparative examples 1 to 5]

Based on the formulation shown in Tables 1 to 6, the skin cleansing agents of Examples 1 to 21 and Comparative examples 1 to 5 were prepared according to a common method.

In the tables, the unit of numerical values is "% by mass", and "-" signifies that the component is not mixed in.

Regarding the prepared skin cleansing agent, [1] foamability, foam retainability, and foam quality, [2] feeling at the time of cleansing and feeling of moistness after cleansing, and [3] preparation stability were evaluated based on the evaluation criteria described below. The evaluation results are shown in Tables 1 to 6.

The details of each of the components shown in Tables 1 to 6 are as follows.
Crystalline cellulose; KG1000 (Ceolus KG1000: trade name, Asahi Kasei Chemicals Corporation) having an average particle size of 50 µm and a ratio of the major axis to the minor axis (L/D) of 3.5
Crystalline cellulose; KG802 (Ceolus KG802: trade name, Asahi Kasei Chemicals Corporation) having an average particle size of 50 µm and a ratio of the major axis to the minor axis (L/D) of 2.8
Crystalline cellulose; pH101 (Ceolus pH101: trade name, Asahi Kasei Chemicals Corporation) having an average particle size of 50 µm and a ratio of the major axis to the minor axis (L/D) of 1.8
Crystalline cellulose; pH102 (Ceolus pH102: trade name, Asahi Kasei Chemicals Corporation) having an average particle size of 90 µm and a ratio of the major axis to the minor axis (L/D) of 1.5
Crystalline cellulose; UF702 (Ceolus UF702: trade name, Asahi Kasei Chemicals Corporation) having an average particle size of 90 µm and a ratio of the major axis to the minor axis (L/D) of 1.5
Crystalline cellulose; UF711 (Ceolus UF711: trade name, Asahi Kasei Chemicals Corporation) having an average particle size of 50 µm and a ratio of the major axis to the minor axis (L/D) of 1.8
Crystalline cellulose; CP102 (Ceolus CP102: trade name, Asahi Kasei Chemicals Corporation) having an average particle size of 106 µm to 212 µm and a ratio of the major axis to the minor axis (L/D) of 1
Crystalline cellulose; CP507 (Ceolus CP507: trade name, Asahi Kasei Chemicals Corporation) having an average particle size of 500 µm to 710 µm and a ratio of the major axis to the minor axis (L/D) of 1

As polyquaternium-7, MERQUAT 550 (trade name) from The Lubrizol Corporation was used.

As glycosyl trehalose, Tornare (trade name) from Hayashibara Co., Ltd. was used.

As kaolin, White Clay (trade name) from MATSUMOTO TRADING Co., Ltd. was used.

### [1] Evaluation of foamability, foam retainability, and foam quality

A 1 v/v% aqueous solution (400 mL) of each of the skin cleansing agents of Examples 1 to 21 and Comparative examples 1 to 5 was prepared and stirred by a homogenizer for 1 minute at 4,500 rpm. Thereafter, the amount and quality of foam immediately after stirring were measured, the amount of foam 10 minutes after stirring was measured, and the foamability, the foam retainability, and the foam quality were evaluated based on the following evaluation criteria. Here, the foam quality was evaluated by visual observation.

### -Evaluation criteria for foamability-

S: The amount of foam was equal to or greater than 1,550 mL.
A: The amount of foam was equal to or greater than 1,500 mL and less than 1,550 mL.
B: The amount of foam was equal to or greater than 1,450 mL and less than 1,500 mL.
C: The amount of foam was less than 1,450 mL.

### -Evaluation criteria for foam retainability-

A: The decrease in the amount of foam 10 minutes after stirring was equal to or less than 3 v/v%.
B: The decrease in the amount of foam 10 minutes after stirring was greater than 3 v/v% and equal to or less than 6 v/v%.
C: The decrease in the amount of foam 10 minutes after stirring was greater than 6 v/v%.

### -Evaluation criteria for foam quality -

A: Fine and uniform foams were formed.
B: Fine foams and medium-sized foams were mixed together.
C: Medium-sized foams and foams larger than the medium-sized foams were observed.

### [2] Panel evaluation (spinnability, feeling at the time of cleansing, and feeling of moistness after cleansing)

Tube-type containers with a hinge cap were filled with each of the skin cleansing agents of Examples 1 to 21 and Comparative examples 1 to 5, thereby preparing facial cleansing foams. Ten professional panelists were asked to actually cleanse their face with the prepared facial cleansing foams and to evaluate the drawing of threads (spinnability) out of the facial cleansing foams at the time of use, the feeling (skin irritation and foreign body sensation) at the time of cleansing, and the feeling of moistness after cleansing according to the following evaluation criteria.

### -Evaluation criteria for spinnability-

A: Two or less out of the ten panelists answered that the container became contaminated because the skin cleansing agent was drawn out into threads.
B: Three to six out of the ten panelists answered that the container became contaminated because the skin cleansing agent was drawn out into threads.
C: Seven or more out of the ten panelists answered that the container became contaminated because the skin cleansing agent was drawn out into threads.

### -Evaluation criteria for feeling at the time of cleansing-

A: None of the ten panelists answered that they experienced skin irritation or had a foreign body sensation.
B: One to four out of the ten panelists answered that they experienced skin irritation or had a foreign body sensation.
C: Five or more out of the ten panelists answered that they experienced skin irritation or had a foreign body sensation.

### -Evaluation criteria for feeling of moistness after cleansing-

A: Seven or more out of the ten panelists answered that they felt refreshed after cleansing and had a feeling of moistness to an appropriate degree.
B: Four to six out of the ten panelists answered that they felt refreshed after cleansing and had a feeling of moistness to an appropriate degree.
C: Three or less out of the ten panelists answered that they felt refreshed after cleansing and had a feeling of moistness to an appropriate degree.

### [3] Temporal stability

Transparent glass containers were filled with each of the skin cleansing agents of Examples 1 to 21 and Comparative examples 1 to 5, and preserved in a thermostatic bath at a temperature of 4°C and a temperature of 35°C respectively for one month. Through hardness evaluation and visual observation, the properties of the skin cleansing agent after preservation were evaluated according to the following criteria.

The hardness of the skin cleansing agent before and after preservation was measured by using a FUDOH Rheometer (trade name, RHEOTECH).

### -Evaluation criteria for hardness-

A: The change in hardness was zero or equal to or less than 30 g.
B: The change in hardness was greater than 30 g and equal to or less than 50 g.
C: The change in hardness was greater than 50 g.

### -Evaluation criteria for visual observation-

A: Phase separation or precipitation was not observed at all.
B: Slight phase separation or precipitation was observed.
C: Phase separation or precipitation was clearly observed.

**[Table 1]**

| (Total amount: 100%) | | | |
|---|---|---|---|
| | | Example 1 | Example 2 |
| KG1000 | Crystalline cellulose | 0.05 | 0.10 |
| CP102 | | - | - |
| CP507 | | - | - |
| Polyquaternium-7 | Polyquaternium-7 | 0.10 | 0.20 |
| Potassium myristate | Higher fatty acid salt | 20.00 | 20.00 |
| Potassium palmitate | | 5.00 | 5.00 |
| Potassium stearate | | 5.00 | 5.00 |
| Potassium laurate | | 3.00 | 3.00 |
| Gycerin | Polyol | 25.00 | 25.00 |
| PEG-6 | | 5.00 | 5.00 |
| PEG-32 | | 5.00 | 5.00 |
| Sorbitol | | 5.00 | 5.00 |
| Glycosyl trehalose | | 2.00 | 2.00 |
| Glyceryl stearate (SE) | Nonionic surfactant | 1.50 | 1.50 |
| Kaolin | Clay | 1.00 | 0.50 |
| Phytosteryl isostearate | Conditioning agent | 0.50 | 0.50 |
| Sodium methyl cocoyl taurate | Cleansing component | 1.00 | 1.00 |
| Hydrolyzed and hydrogenated starch | Moisturizing component | 1.15 | 1.15 |
| Colorant | | Appropriate amount | Appropriate amount |
| Fragrance | | Appropriate amount | Appropriate amount |
| Preservative | Preservative Appropriate | Appropriate amount | Appropriate amount |
| Antioxidant | | Appropriate amount | Appropriate amount |
| Water | | Balance | Balance |
| Foamability foam retainability foam quality | Foamability | A | A |
| | Foam retainability | A | A |
| | Foam quality | A | A |
| Panel evaluation | Spinnability | B | B |
| | Feeling at the time of cleansing | A | A |
| | Feeling of moistness after cleansing | A | A |
| Temporal stability | Change in hardness | A | A |
| | Visual observation | A | A |

**[Table 2]**

| (Total amount: 100%) | | | | | | |
|---|---|---|---|---|---|---|
| | | Comparative example 1 | Comparative example 2 | Comparative example 3 | Comparative example 4 | Comparative example 5 |
| KG1000 | Crystalline cellulose | - | - | - | - | 0.10 |
| CP102 | | - | - | 1.00 | - | - |
| CP507 | | - | - | - | 1.00 | - |
| Polyquaternium-7 | Polyquaternium-7 | 0.10 | 0.20 | 0.10 | 0.10 | - |
| Potassium myristate | Higher fatty acid salt | 20.00 | 20.00 | 20.00 | 20.00 | 20.00 |
| Potassium palmitate | | 5.00 | 5.00 | 5.00 | 5.00 | 5.00 |
| Potassium stearate | | 5.00 | 5.00 | 5.00 | 5.00 | 5.00 |
| Potassium laurate | | 3.00 | 3.00 | 3.00 | 3.00 | 3.00 |
| Gycerin | Polyol | 25.00 | 25.00 | 25.00 | 25.00 | 25.00 |
| PEG-6 | | 5.00 | 5.00 | 5.00 | 5.00 | 5.00 |
| PEG-32 | | 5.00 | 5.00 | 5.00 | 5.00 | 5.00 |
| Sorbitol | | 5.00 | 5.00 | 5.00 | 5.00 | 5.00 |
| Glycosyl trehalose | | 2.00 | 2.00 | 2.00 | 2.00 | 2.00 |
| Glyceryl stearate (SE) | Nonionic surfactant | 1.50 | 1.50 | 1.50 | 1.50 | 1.50 |
| Kaolin | Clay | 1.00 | 0.50 | 1.00 | 1.00 | 0.50 |
| Phytosteryl isostearate | Conditioning agent | 0.50 | 0.50 | 0.50 | 0.50 | 0.50 |
| Sodium methyl cocoyl taurate | Cleansing component | 1.00 | 1.00 | 1.00 | 1.00 | 1.00 |
| Hydrolyzed and hydrogenated starch | Moisturizing component | 1.15 | 1.15 | 1.15 | 1.15 | 1.15 |
| Colorant | | Appropriate amount | Appropriate amount | Appropriate amount | Appropriate amount | Appropriate amount |
| Fragrance | | Appropriate amount | Appropriate amount | Appropriate amount | Appropriate amount | Appropriate amount |
| Preservative | | Appropriate amount | Appropriate amount | Appropriate amount | Appropriate amount | Appropriate amount |
| Antioxidant | | Appropriate amount | Appropriate amount | Appropriate amount | Appropriate amount | Appropriate amount |
| Water | | Balance | Balance | Balance | Balance | Balance |
| Foamability foam retainability foam quality | Foamability | B | B | A | A | A |
| | Foam retainability | A | A | C | C | C |
| | Foam quality | C | B | C | C | B |
| Panel evaluation | Spinnability | C | C | C | C | A |
| | Feeling at the time of cleansing | A | A | B | B | A |
| | Feeling of moistness after cleansing | B | A | C | C | C |
| Temporal stability | Change in hardness | B | B | C | C | B |
| | Visual observation | A | A | C | C | A |

**[Table 3]**

| (Total amount: 100%) | | | | | | | |
|---|---|---|---|---|---|---|---|
| | | Example 3 | Example 4 | Example 5 | Example 6 | Example 7 | Example 8 |
| KG1000(50) | Crystalline cellulose (average particle size: µm) | 1.00 | - | - | - | - | - |
| KG802(50) | | - | 1.00 | - | - | - | - |
| pH101(50) | | - | - | 1.00 | - | - | - |
| pH102(90) | | - | - | - | 1.00 | - | - |
| UF702(90) | | - | - | - | - | 1.00 | - |
| UF711(50) | | - | - | - | - | - | 1.00 |
| Polyquaternium-7 | Polyquaternium-7 | 0.10 | 0.10 | 0.10 | 0.10 | 0.10 | 0.10 |
| Potassium myristate | Higher fatty acid salt | 20.00 | 20.00 | 20.00 | 20.00 | 20.00 | 20.00 |
| Potassium palmitate | | 5.00 | 5.00 | 5.00 | 5.00 | 5.00 | 5.00 |
| Potassium stearate | | 5.00 | 5.00 | 5.00 | 5.00 | 5.00 | 5.00 |
| Potassium laurate | | 3.00 | 3.00 | 3.00 | 3.00 | 3.00 | 3.00 |
| Glycerin | Polyol | 25.00 | 25.00 | 25.00 | 25.00 | 25.00 | 25.00 |
| PEG-6 | | 5.00 | 5.00 | 5.00 | 5.00 | 5.00 | 5.00 |
| PEG-32 | | 5.00 | 5.00 | 5.00 | 5.00 | 5.00 | 5.00 |
| Sorbitol | | 5.00 | 5.00 | 5.00 | 5.00 | 5.00 | 5.00 |
| Glycosyl trehalose | | 2.00 | 2.00 | 2.00 | 2.00 | 2.00 | 2.00 |
| Glyceryl stearate (SE) | Nonionic surfactant | 1.50 | 1.50 | 1.50 | 1.50 | 1.50 | 1.50 |
| Kaolin | Clay | 1.00 | 1.00 | 1.00 | 1.00 | 1.00 | 1.00 |
| Phytosteryl isostearate | Conditioning agent | 0.50 | 0.50 | 0.50 | 0.50 | 0.50 | 0.50 |
| Sodium methyl cocoyl taurate | Cleansing component | 1.00 | 1.00 | 1.00 | 1.00 | 1.00 | 1.00 |
| Hydrolyzed and hydrogenated starch | Moisturizing component | 1.15 | 1.15 | 1.15 | 1.15 | 1.15 | 1.15 |
| Colorant | | Appropriate amount | Appropriate amount | Appropriate amount | Appropriate amount | Appropriate amount | Appropriate amount |
| Fragrance | | Appropriate amount | Appropriate amount | Appropriate amount | Appropriate amount | Appropriate amount | Appropriate amount |
| Preservative | | Appropriate amount | Appropriate amount | Appropriate amount | Appropriate amount | Appropriate amount | Appropriate amount |
| Antioxidant | | Appropriate amount | Appropriate amount | Appropriate amount | Appropriate amount | Appropriate amount | Appropriate amount |
| Water | | Balance | Balance | Balance | Balance | Balance | Balance |
| Foamability·foam retainability·foam quality | Foamability | S | A | B | B | B | B |
| | Foam retainability | A | A | A | B | B | A |
| | Foam quality | A | A | B | B | B | B |
| Panel evaluation | Spinnability | A | A | B | B | B | B |
| | Feeling at the time of cleansing | A | A | A | A | A | A |
| | Feeling of moistness after cleansing | A | A | A | A | A | A |
| Temporal stability | Change in hardness | B | B | B | B | B | B |
| | Visual observation | A | A | A | A | A | A |

**[Table 4]**

| (Total amount: 100%) | | | | | | | |
|---|---|---|---|---|---|---|---|
| | | Example 9 | Example 10 | Example 11 | Example 12 | Example 13 | Example 14 |
| KG1000 | Crystalline cellulose | 0.10 | 0.10 | 0.10 | 0.10 | 0.10 | 0.10 |
| Polyquaternium-7 | Polyquaternium-7 | 0.10 | 0.10 | 0.10 | 0.10 | 0.10 | 0.10 |
| Potassium myristate | Higher fatty acid salt | 20.00 | 20.00 | 20.00 | 20.00 | 25.00 | 25.00 |
| Potassium palmitate | | 5.00 | 5.00 | 5.00 | 5.00 | 5.00 | - |
| Potassium stearate | | 5.00 | 5.00 | 5.00 | 5.00 | - | 5.00 |
| Potassium laurate | | 3.00 | 3.00 | 3.00 | 3.00 | 3.00 | 3.00 |
| Glycerin | Polyol | 25.00 | 25.00 | 25.00 | 15.00 | 25.00 | 25.00 |
| BG | | | - | - | 10.00 | - | - |
| PEG-6 | | 5.00 | 5.00 | 5.00 | 5.00 | 5.00 | 5.00 |
| PEG-32 | | 5.00 | 5.00 | 5.00 | 5.00 | 5.00 | 5.00 |
| Sorbitol | | 5.00 | 5.00 | 5.00 | 5.00 | 5.00 | 5.00 |
| Glycosyl trehalose | | 2.00 | 2.00 | 2.00 | - | 2.00 | 2.00 |
| Glyceryl glucoside | | - | - | - | 2.00 | - | - |
| Glyceryl stearate (SE) | Nonionic surfactant | - | 3.00 | 1.50 | 1.50 | 1.50 | 1.50 |
| Kaolin | Clay | 0.50 | 0.50 | - | 0.50 | 0.50 | 0.50 |
| Talc | | - | - | 0.50 | - | - | - |
| Phytosteryl isostearate | Conditioning agent | 0.50 | 0.50 | 0.50 | 0.50 | 0.50 | 0.50 |
| Sodium methyl cocoyl taurate | Cleansing component | 1.00 | 1.00 | 1.00 | 1.00 | 1.00 | 1.00 |
| Hydrolyzed and hydrogenated starch | Moisturizing component | 1.15 | 1.15 | 1.15 | - | 1.15 | 1.15 |
| Fermented broth of lactobacillus/European pear juice | | - | - | - | 0.10 | - | - |
| Blackberry lily extract | | - | - | - | 0.10 | - | - |
| Bilberry leaf extract | | - | - | - | 0.10 | - | - |
| Artichoke leaf extract | | - | - | - | 0.10 | - | - |
| Stearyl glycyrrhetinate | Anti-inflammatory component | - | - | - | 0.10 | - | - |
| Tocopherol acetate | | - | - | - | 0.10 | - | - |
| Colorant | | Appropriate amount | Appropriate amount | Appropriate amount | Appropriate amount | Appropriate amount | Appropriate amount |
| Fragrance | | Appropriate amount | Appropriate amount | Appropriate amount | Appropriate amount | Appropriate amount | Appropriate amount |
| Preservative | | Appropriate amount | Appropriate amount | Appropriate amount | Appropriate amount | Appropriate amount | Appropriate amount |
| Antioxidant | | Appropriate amount | Appropriate amount | Appropriate amount | Appropriate amount | Appropriate amount | Appropriate amount |
| Water | | Balance | Balance | Balance | Balance | Balance | Balance |
| Foamability·foam | Foamability | A | A | A | A | S | S |
| retainability·foam | Foam retainability | B | A | A | A | A | A |
| quality | Foam quality | A | A | A | A | A | A |
| Panel evaluation | Spinnability | B | B | B | B | B | B |
| | Feeling at the time of cleansing | A | A | A | A | A | A |
| | Feeling of moistness after cleansing | A | A | A | A | A | A |
| Temporal stability | Change in hardness | B | B | B | B | B | B |
| | Visual observation | B | A | A | A | B | B |

**[Table 5]**

| (Total amount: 100%) | | | | | |
|---|---|---|---|---|---|
| | | Example 15 | Example 16 | Example 17 | Example 18 |
| KG1000 | Crystalline cellulose | 0.10 | 0.10 | 0.10 | 0.10 |
| Polyquaternium-7 | Polyquaternium-7 | 0.10 | 0.10 | 0.10 | 0.10 |
| Potassium myristate | Higher fatty acid salt | 20.00 | 20.00 | 20.00 | 20.00 |
| Potassium palmitate | | 5.00 | 5.00 | 5.00 | 5.00 |
| Potassium stearate | | 5.00 | 5.00 | 5.00 | 5.00 |
| Potassium laurate | | 3.00 | 3.00 | 3.00 | 3.00 |
| Glycerin | Polyol | 25.00 | 25.00 | 25.00 | 25.00 |
| PEG-6 | | 5.00 | 5.00 | 5.00 | 5.00 |
| PEG-32 | | 5.00 | 5.00 | 5.00 | 5.00 |
| Sorbitol | | 5.00 | 5.00 | 5.00 | 5.00 |
| Glycosyl trehalose | | 2.00 | 2.00 | 2.00 | 2.00 |
| Glyceryl stearate (SE) | Nonionic surfactant | 1.50 | 1.50 | 1.50 | 1.50 |
| Kaolin | Clay | 0.50 | 0.50 | 0.50 | 0.50 |
| Phytosteryl isostearate | Conditioning agent | - | 2.00 | - | - |
| Di(phytosteryl/octyldodecyl) lauroyl glutamate | | - | - | 0.50 | - |
| Jojoba seed oil | | - | - | - | 0.50 |
| Shea fat and oil | | | - | - | - |
| Macadamia nut oil | | - | - | - | - |
| Vaseline | | - | - | - | - |
| Sodium methyl cocoyl taurate | Cleansing component | 1.00 | 1.00 | 1.00 | 1.00 |
| Hydrolyzed and hydrogenated starch | Moisturizing component | 1.15 | 1.15 | 1.15 | 1.15 |
| Colorant | | Appropriate amount | Appropriate amount | Appropriate amount | Appropriate amount |
| Fragrance | | Appropriate amount | Appropriate amount | Appropriate amount | Appropriate amount |
| Preservative | | Appropriate amount | Appropriate amount | Appropriate amount | Appropriate amount |
| Antioxidant | | Appropriate amount | Appropriate amount | Appropriate amount | Appropriate amount |
| Water | | Balance | Balance | Balance | Balance |
| Foamability·foam retainability·foam quality | Foamability | A | A | S | S |
| | Foam retainability | A | B | A | B |
| | Foam quality | A | A | A | A |
| Panel evaluation | Spinnability | B | B | B | B |
| | Feeling at the time of cleansing | A | A | A | A |
| | Feeling of moistness after cleansing | B | A | A | A |
| Temporal stability | Change in hardness | B | B | B | B |
| | Visual observation | A | A | B | A |

**[Table 6]**

| (Total amount: 100%) | | | | |
|---|---|---|---|---|
| | | Example 19 | Example 20 | Example 21 |
| KG1000 | Crystalline cellulose | 0.10 | 0.10 | 0.10 |
| Polyquaternium-7 | Polyquaternium-7 | 0.10 | 0.10 | 0.10 |
| Potassium myristate | Higher fatty acid salt | 20.00 | 20.00 | 20.00 |
| Potassium palmitate | | 5.00 | 5.00 | 5.00 |
| Potassium stearate | | 5.00 | 5.00 | 5.00 |
| Potassium laurate | | 3.00 | 3.00 | 3.00 |
| Glycerin | Polyol | 25.00 | 25.00 | 25.00 |
| PEG-6 | | 5.00 | 5.00 | 5.00 |
| PEG-32 | | 5.00 | 5.00 | 5.00 |
| Sorbitol | | 5.00 | 5.00 | 5.00 |
| Glycosyl trehalose | | 2.00 | 2.00 | 2.00 |
| Glyceryl stearate (SE) | Nonionic surfactant | 1.50 | 1.50 | 1.50 |
| Kaolin | Clay | 0.50 | 0.50 | 0.50 |
| Phytosteryl isostearate | Conditioning agent | - | - | - |
| Di(phytosteryl/octyldodecyl) lauroyl glutamate | | - | - | - |
| Jojoba seed oil | | - | - | - |
| Shea fat and oil | | 0.50 | - | - |
| Macadamia nut oil | | - | 0.50 | - |
| Vaseline | | - | - | 0.50 |
| Sodium methyl cocoyl taurate | Cleansing component | 1.00 | 1.00 | 1.00 |
| Hydrolyzed and hydrogenated starch | Moisturizing component | 1.15 | 1.15 | 1.15 |
| Colorant | | Appropriate amount | Appropriate amount | Appropriate amount |
| Fragrance | | Appropriate amount | Appropriate amount | Appropriate amount |
| Preservative | | Appropriate amount | Appropriate amount | Appropriate amount |
| Antioxidant | | Appropriate amount | Appropriate amount | Appropriate amount |
| Water | | Balance | Balance | Balance |
| Foamability·foam retainability·foam quality | Foamability | S | S | S |
| | Foam retainability | A | B | A |
| | Foam quality | A | A | A |
| Panel evaluation | Spinnability | B | B | B |
| | Feeling at the time of cleansing | A | A | A |
| | Feeling of moistness after cleansing | A | A | A |
| Temporal stability | Change in hardness | A | B | B |
| | Visual observation | A | A | A |

As is evident from Tables 1 to 6, the skin cleansing agents of the examples exhibited excellent foamability, are inhibited from leaving a feeling of tightness on the skin after cleansing and inhibited from contaminating the container by being drawn out into threads at the time of use, exhibit excellent foam retainability and foam quality, and are excellent in the temporal stability as a skin cleansing agent.

## Claims

1. A skin cleansing agent comprising:
crystalline cellulose having an average particle size of equal to or less than 100 µm;
polyquaternium-7;
a higher fatty acid salt having 8 to 22 carbon atoms; and
a polyol.

2. The skin cleansing agent according to claim 1,
wherein a ratio of a major axis to a minor axis of the crystalline cellulose is equal to or greater than 1.5.

3. The skin cleansing agent according to claim 1 or 2, further comprising clay.

4. The skin cleansing agent according to any one of claims 1 to 3, further comprising a nonionic surfactant having a linear fatty acid ester structure having 8 to 22 carbon atoms.

5. The skin cleansing agent according to any one of claims 1 to 4, further comprising a conditioning agent.

6. The skin cleansing agent according to claim 1, wherein the crystalline cellulose has an average particle size of from 20 µm to 100 µm.

7. The skin cleansing agent according to claim 1, wherein a content of the crystalline cellulose is from 0.01% by mass to 10% by mass with respect to a total mass of the skin cleansing agent.

8. The skin cleansing agent according to claim 1, wherein a content of the polyquaternium-7 is from 0.01% by mass to 5% by mass with respect to a total mass of the skin cleansing agent.

9. The skin cleansing agent according to claim 1, wherein a content ratio of the crystalline cellulose to the polyquaternium-7 is from 1:5 to 20:1 based on mass.

10. The skin cleansing agent according to claim 1, wherein a content of the higher fatty acid salt is from 15% by mass to 60% by mass with respect to a total mass of the skin cleansing agent.

11. The skin cleansing agent according to claim 1, wherein the polyol is selected from the group consisting of glycerin, 1,3-butylene glycol, a polyethylene glycol having an average molecular weight of equal to or less than 10,000, glycosyl trehalose, sorbitol, and glyceryl glucoside.

12. The skin cleansing agent according to claim 1, wherein a content of the polyol is from 5% by mass to 60% by mass with respect to a total mass of the skin cleansing agent.

## Patentansprüche

1. Hautreinigungsmittel aufweisend:
Kristalline Cellulose mit einer mittleren Partikelgröße von gleich oder weniger als 100 µm;
Polyquaternium-7;
ein Salz einer höheren Fettsäure mit 8 bis 22 Kohlenstoffatomen; und
ein Polyol.

2. Hautreinigungsmittel nach Anspruch 1, bei dem das Verhältnis einer Hauptachse zu einer Nebenachse der kristallinen Cellulose gleich oder größer als 1,5 ist.

3. Hautreinigungsmittel nach Anspruch 1 oder 2, außerdem Ton aufweisend.

4. Hautreinigungsmittel nach einem der Ansprüche 1 bis 3, außerdem ein nichtionisches oberflächenaktives Mittel mit der Struktur eines Esters einer linearen Fettsäure mit 8 bis 22 Kohlenstoffatomen aufweisend.

5. Hautreinigungsmittel nach einem der Ansprüche 1 bis 4, außerdem ein Konditionierungsmittel aufweisend.

6. Hautreinigungsmittel nach Anspruch 1, bei dem die kristalline Cellulose eine mittlere Partikelgröße von 20 µm bis 100 µm hat.

7. Hautreinigungsmittel nach Anspruch 1, bei dem der Gehalt an der kristallinen Cellulose von 0,01 Masseprozent bis 10 Masseprozent, bezogen auf die Gesamtmasse des Hautreinigungsmittels, beträgt.

8. Hautreinigungsmittel nach Anspruch 1, bei dem der Gehalt an dem Polyquaternium-7 von 0,01 Masseprozent bis 5 Masseprozent, bezogen auf die Gesamtmasse des Hautreinigungsmittels, beträgt.

9. Hautreinigungsmittel nach Anspruch 1, bei dem das Verhältnis des Gehalts an der kristallinen Cellulose zu dem Gehalt an dem Polyquaternium-7 von 1:5 bis 20:1, auf Massebasis, beträgt.

10. Hautreinigungsmittel nach Anspruch 1, bei dem der Gehalt an dem Salz der höheren Fettsäure von 15 Masseprozent bis 60 Masseprozent, bezogen auf die Gesamtmasse des Hautreinigungsmittels, beträgt.

11. Hautreinigungsmittel nach Anspruch 1, bei dem das Polyol ausgewählt ist aus der Gruppe, die aus Glycerin, 1,3-Butylenglycol, einem Polyethylenglycol mit einem mittleren Molekulargewicht von gleich oder weniger als 10.000, Glycosyltrehalose, Sorbitol und Glycerylglucosid besteht.

12. Hautreinigungsmittel nach Anspruch 1, bei dem der Gehalt an dem Polyol von 5 Masseprozent bis 60 Masseprozent, bezogen auf die Gesamtmasse des Hautreinigungsmittels, beträgt.

## Revendications

1. Agent nettoyant pour la peau, comprenant :
de la cellulose cristalline présentant une taille de particules moyenne inférieure ou égale à 100 µm ;
du polyquaternium-7 ;
un sel d'acides gras supérieurs présentant de 8 à 22 atomes de carbone, et
un polyol.

2. Agent nettoyant pour la peau selon la revendication 1,
dans lequel un rapport d'un grand axe à un petit axe de la cellulose cristalline est supérieur ou égal à 1,5.

3. Agent nettoyant pour la peau selon la revendication 1 ou 2, comprenant en outre de l'argile.

4. Agent nettoyant pour la peau selon l'une quelconque des revendications 1 à 3, comprenant en outre un tensioactif non ionique présentant une structure d'ester d'acides gras linéaire présentant de 8 à 22 atomes de carbone.

5. Agent nettoyant pour la peau selon l'une quelconque des revendications 1 à 4, comprenant en outre un agent conditionneur.

6. Agent nettoyant pour la peau selon la revendication 1, dans lequel la cellulose cristalline présente une taille de particules moyenne allant de 20 µm à 100 µm.

7. Agent nettoyant pour la peau selon la revendication 1, dans lequel une teneur de la cellulose cristalline est comprise dans une plage allant de 0,01 % en masse à 10 % en masse par rapport à une masse totale de l'agent nettoyant pour la peau.

8. Agent nettoyant pour la peau selon la revendication 1, dans lequel une teneur du polyquaternium-7 est comprise dans une plage allant de 0,01 % en masse à 5 % en masse par rapport à une masse totale de l'agent nettoyant pour la peau.

9. Agent nettoyant pour la peau selon la revendication 1, dans lequel un rapport des teneurs entre la cellulose cristalline et le polyquaternium-7 est compris dans une plage allant de 1 : 5 à 20 : 1 sur la base de la masse.

10. Agent nettoyant pour la peau selon la revendication 1, dans lequel une teneur du sel d'acides gras supérieurs est comprise dans une plage allant de 15 % en masse à 60 % en masse par rapport à une masse totale de l'agent nettoyant pour la peau.

11. Agent nettoyant pour la peau selon la revendication 1, dans lequel le polyol est sélectionné parmi le groupe consistant en la glycérine, le 1,3-butylèneglycol, un polyéthylèneglycol présentant un poids moléculaire moyen inférieur ou égal à 10 000, du glycosyl tréhalose, du sorbitol et du glycosyl glucoside.

12. Agent nettoyant pour la peau selon la revendication 1, dans lequel une teneur du polyol est comprise dans une plage allant de 5 % en masse à 60 % en masse par rapport à une masse totale de l'agent nettoyant pour la peau.
